# EUROPEAN PATENT APPLICATION

(11) **EP 4 668 284 A1**
(43) Date of publication of application: **24.12.2025**
(21) Application number: 24183811.9
(22) Date of filing: 21.06.2024
(51) Int. Cl.: G16H 15/00, G16H 50/20, G16H 50/70

(54) **SYSTEM FOR ACQUIRING A CURRENT MEDICAL IMAGE OF A PATIENT AND GENERATING A CURRENT FINAL REPORT BASED ON THE ACQUIRED CURRENT MEDICAL IMAGE, COMPUTER PROGRAM PRODUCT, AND METHOD FOR USING THE SYSTEM**

(71) Applicant: Siemens Healthineers AG, 91301 Forchheim (DE)
(72) Inventor: KOLLERATHU, Varghese, 560066 Whitefield, Bengaluru, Karnataka (IN); FARHAND, Sepehr, 19355 Malvern, Chester (US); RAMESH, Rahul, 560064 Avalahalli, Bengaluru (IN)
(74) Representative: HKW Intellectual Property PartG mbB

(57) **Abstract**

A system for acquiring a current medical image of a patient and generating a current final report based on the acquired current medical image, the system comprising:
a graphical user interface for receiving and displaying the current medical image as acquired by a medical imaging unit, and for providing a selection of a number N1 of findings within the acquired current medical image by a user of the system, with N1 ≥ 1,
a database storing prior examination data,
a content-based image retrieval system for comparing the N1 findings with a selection of the prior examination data, and for determining a current pathological condition for each of the N1 findings based on a result of the comparison and the selection of the prior examination data,
a data collector unit for summarizing all the current pathological conditions and the selection of the prior examination data into a data set, and
a large language model for generating the current final report based on the data set,
wherein the graphical user interface is configured to allow a modification of the data set by the user, and
wherein the system is configured to extend, based on the data set, the database and/or training data of the content-based image retrieval system.

## Description

The present invention relates to a system for acquiring a current medical image of a patient and generating a current final report based on the acquired current medical image, a method for using such a system and a computer program product.

Medical imaging plays an important role in the diagnosis and treatment of cancer or other diseases. Modalities such as radiography, computed tomography, or magnetic resonance imaging are used to acquire a current medical image which is analyzed to detect abnormalities, for example a lesion or nodule. Nonetheless, in a medical workflow, reporting on findings within the current medical image takes disproportionally more time than acquiring the current medical image. As the role of imaging in the healthcare space is on the rise, the number of cases a radiologist needs to report on a day-to-day basis is one the rise. Literature suggests that in this environment, the detection rate of the radiologist drops as high as 40%, resulting in wrong diagnosis and missed findings. In addition, the delay associated with obtaining the current final report can lead to a longer stay in the hospital and to increased expenses. Independent of the grammatical term usage, individuals with male, female or other gender identities are included within the term.

Hence, there is a need to trim down the time required to generate the current final report based on the current medical image. CAD-based tools may facilitate this goal. However, according to internal knowledge, most radiologists prefer the usage of generic tools such as adding a distance line to the current medical image to measure a dimension of an abnormality in the current medical image, the distance to another anatomical feature visible in the image, or the like.

Therefore, it is one objective of the present invention to improve the acquiring of the current medical image and the generating of the current final report.

According to a first aspect, a system for acquiring a current medical image of a patient and generating a current final report based on the acquired current medical image is provided. The system comprises:
a graphical user interface for receiving and displaying the current medical image as acquired by a medical imaging unit, and for providing a selection of a number N1 of findings within the acquired current medical image by a user of the system, with N1 ≥ 1,
a database storing prior examination data,
a content-based image retrieval system for comparing the N1 findings with a selection of the prior examination data, and for determining a current pathological condition for each of the N1 findings based on a result of the comparison and the selection of the prior examination data,
a data collector unit for summarizing all the current pathological conditions and the selection of the prior examination data into a data set, and
a large language model for generating the current final report based on the data set,
wherein the graphical user interface is configured to allow a modification of the data set by the user, and
wherein the system is configured to extend, based on the data set, the database and/or training data of the content-based image retrieval system.

The current medical image can be the result of an X-ray examination, a mammography examination, a magnetic resonance tomography (MRT), a computed tomography (CT), or the like. The current medical image can be a two-dimensional or three-dimensional representation of at least a section of the patient. Hereby, the current medical image is acquired during a current examination with the patient. It is also possible to acquire a plurality of current medical images within the same examination, for example showing multiple slices at multiple locations in relation to the patient. Therefore, when it is referred to the current medical image, the current medical image can represent a plurality of current medical images as well. The N1 findings can be marked by the user within the current medical image, wherein, in this context, each of the N1 findings can be understood as any object, structure or component within the body of the patient. In other words, the N1 findings represent something of interest to the user and can both show an abnormality within the patient's body or a normal condition. Within the current context, the user can be a technician and/or a radiologist.

The graphical user interface can allow the user to annotate any relevant feature in the current medical image. For example, the graphical user interface can allow the user to add a distance line to the image to indicate a dimension of one of the N1 findings, the distance to another anatomical feature visible in the current medical image, or such. These annotations can be stored together with the current medical image and the current final report in a current patient record.

The prior examination data can comprise a plurality of prior patient records of prior examinations of the patient and/or other patients, each prior patient record comprising one or more prior medical images and a correlated prior final report. The selection of the prior examination data can represent a single or a plurality of the prior patient records.

The term "comparing" can be understood as evaluating anatomical similarities and/or differences between different medical images, in particular between the current medical image and one of the prior medical images. For example, a possible growth of an anatomical structure or an abnormality between two different medical images can be evaluated. In particular, the content-based image retrieval system is configured to compare the N1 findings within the current medical image with data associated to prior medical images of the prior patient data using a cosine distance. Other manners for performing the comparison are feasible as well. If a similarity between a certain one of the N1 findings and the data of a certain one of the prior patient records is above a certain threshold, the current pathological condition of the certain one of the N1 findings is associated with a prior pathological condition of the certain prior patient record, providing the current pathological condition.

In the present context, the current pathological condition can be understood as the medical meaning of a certain one of the N1 findings. For example, a current pathological condition can be a specific disease, for example lung cancer. The content-based image retrieval system is configured to determine the current pathological condition for each of the N1 findings based on the result of the comparison and the selection of the prior examination data. For example, the prior examination data can comprise a prior medical image showing a certain structure within the body of the patient and a correlated prior final report which defines the structure as being in healthy condition. By comparing the N1 findings with said prior medical image, a possible variation of the structure can be identified, causing the content-based image retrieval system to determine a possible existence of a specific disease, for example lung cancer. The possible variations of the structure as identified by the content-based image retrieval system can be a growth of the structure, a varying shape of the structure, for example, changing from a speculated shape into a rounded shape, or the transition of a margin of a mass from a well-defined margin into an irregular margin. In this way, for each of the N1 findings, the corresponding current pathological condition is determined. To sum up, the content-based image retrieval system determines the current pathological condition on both a comparison between the current medical image and at least one prior medical image, as well as based on a prior pathological condition corresponding to the prior medical image. Hence, a prior diagnosis is respected for determining a current diagnosis.

All available data correlated to the current examination of the patient, including the current medical image with all the N1 findings, and all the current pathological conditions, possibly augmented by the prior pathological conditions and the correlated prior final reports, are summarized into the data set by the data collector unit.

The graphical user interface can be configured to allow the modification of the data set by the user. The modification may comprise both an editing of existing data within the data set and/or adding new information to the data set. For example, the current pathological condition as determined by the content-based image retrieval system can be changed manually by the user. This way, possible mistakes can be corrected by the user. Furthermore, the user can add supplementary data correlated to the N1 findings, for example further specifying information, annotations, and/or remarks.

The large language model may comprise a deep neural network employing, for example, a transformer architecture, and having a large number of neurons, such as one billion neurons or more, preferable 7 billion neurons or more, more preferably 13 billion of neurons or more, and most preferably 70 billion neurons or more. In particular, the large language model may be configured to receive a sequence of tokens, such as words, syllables, or lexemes, as input and to output one token as output that is deemed to be the "most probable" next token. In particular, the large language model may be operated in a generative or autoregressive manner, that is: an entry being a sequence of tokens may be provided to the large language model as input, the first most probable next token output by the large language model may be appended to the entry to obtain a new entry to be provided to the large language model as input in the next iteration, and by repeating this sequence of iterations, the large language model may generate a meaningful most probable response to the entry that comprises several sentences or even paragraphs.

In particular, the large language model can be pre-trained so as to understand natural language and have the capability to give meaningful responses about an arbitrary subject. Such pre-training is possible by using a text database that is the result from crawling a portion, preferably a substantial portion, of the Internet as language training data. Herein, any given text comprising N >= 2 words may provide up to N-1 language training datasets, each language training dataset comprising the first n<N words from the text as language input training data and the n+1-th word of the text as language output training data, for n=1 ... N-1. As such, no labelling of the language training data used for the pre-training is required. However, the pre-training of the large language model may, optionally, also comprise steps of supervised training using labelled language training data generated by humans so as to further improve the capability of the large language model to provide meaningful responses to the user.

Examples for a pre-trained generative large language model include OpenAI's ChatGPT 3 and 4, Google AI's PaLM, DeepMind's Chinchilla, and Meta's LLaMa and LLaMA 2 models. The latter pre-trained large language models from Meta are available for download from the Internet, while the former pre-trained models can be accessed and licensed on the Internet, including the possibility to perform further custom fine-tuning.

In particular, the large language model can be fine-tuned for the tasks to which it is going to be applied according to the proposed solution. That is, fine-tuning may comprise unsupervised or supervised further training of the large language model, using language training data that relates to the suggested system.

The large language model can be used for generating the current final report based on the data set. Hence, the summarized information within the data set can be transformed into the current final report, representing a textual representation of the identified N1 findings and the determined current pathological conditions. In particular, the current final report can be generated automatically, without requiring further user input. The current final report can replace the conventional report written manually and/or dictated by the user.

The system is configured to extend, based on the modification of the data set, the database and/or the training data of the content-based image retrieval system. Preferably, all the information of the data set is used to extend the database and/or the training data. The training data can be used to train the content-based image retrieval system. In particular, in case the content-based image retrieval system comprises a machine learning algorithm, the training data can be used for supervised and/or unsupervised training of the machine learning algorithm. To extend the database means that the current patient record, comprising the current medical image, the N1 findings, all the pathological conditions, and/or the current final report, is stored in the database.

In a beneficial manner, the suggested system enables the automatic generation of the current final report based on the M1 findings, thus saving time, and reducing the risk of errors correlated to a diagnosis concerning the current medical image. In addition, the extension of the database and/or the training data enables a continuous improvement of the system, in particular of the content-based image retrieval system, thus continuously improving the precision of the determination of the current pathological condition. Last but not least, by facilitating information of the prior examination data, historical data can be used to further improve the precision of the determination of the current pathological condition.

According to an embodiment, the content-based image retrieval system comprises an anomaly detector for detecting an anomaly within each of the N1 findings. In particular, the anomaly detector is configured to set the respective current pathological condition to a healthy state in case no anomaly is detected.

The anomaly detector can comprise a further machine learning algorithm which can be trained to detect an anomaly within each of the N1 findings. In particular, each of the N1 findings can be categorized as representing an anomaly in case the anatomical structure corresponding to the respective one of the N1 findings shows certain properties including size, shape, density, density distribution, a temporal variation of the afore-mentioned, or the like. In case the anomaly detector regards a certain one of the N1 findings as representing an anatomical structure without an anomaly, the corresponding current pathological condition can be set to a healthy state. For the certain one of the N1 findings, the comparison with prior examination data can be omitted and, thus, the process of generating the current final report be accelerated.

According to a further embodiment, the content-based image retrieval system comprises a comparison unit for comparing the N1 findings with the selection of the prior examination data.

The comparison unit is one component of the content-based image retrieval system and performs the comparison of the N1 findings with the selection of the prior examination data. In particular, the comparison unit is configured to compare each of the N1 findings with prior medical images and/or data corelated to prior medical images, wherein the prior medical images are stored in the database and allocated to prior medical records of the patient and/or at least one of a plurality of further patients. The comparison unit can comprise a machine learning algorithm which is continuously trained during usage of the system by the training data. Subdividing the content-based image retrieval system can be beneficial in terms of a function-oriented design of the content-based image retrieval system.

According to a further embodiment, the content-based image retrieval system comprises a foundation model for determining one fingerprint for each of the N1 findings, and for determining a number N2 of fingerprints for the selection of the prior examination data, with N2 ≥ 1, wherein the comparison unit is configured to compare the N1 fingerprints for the N1 findings with the N2 fingerprints for the selection of the prior examination data.

In the present context, each fingerprint can be understood to be a vector and/or an array of features correlated to a certain anatomical structure and/or to a certain medical image. Hereby, each fingerprint can include a length, a diameter, a cross-section, a volume, a density, a mass, a volume distribution, a mass distribution, or the like. Each fingerprint can further comprise the anatomical location of the respective anatomical structure. The term "fingerprint" may also be referred to as signature or higher-order features. Generally, when it is referred to comparing a finding or a medical image, it can be understood as comparing the respective fingerprint of the finding or of the medical image, respectively. Hence, the comparison unit can be configured to compare a fingerprint of each of the N1 findings with one or more fingerprints correlated to the selection of the prior examination data. In particular, in order to compare the N1 findings with the selection of the prior examination data, the N1 fingerprints for the N1 findings are compared with the N2 fingerprints for the selection of the prior examination data. For example, a length or a volume of the N1 fingerprints for the N1 findings is compared to a length or a volume of the N2 fingerprints allocated to the selection of the prior examination data. The foundation model comprises a machine learning algorithm which is continuously trained during usage of the system by the training data. In a beneficial manner, the comparison of respective fingerprints increases the reliability and the precision of the comparison. In addition, medical images of varying quality, image size, different point of view, and/or different acquisition manner can be compared with one another by comparing the respective fingerprints instead of merely comparing "pixels" and/or "voxels" of different medical images with one another.

According to a further embodiment, the selection of the prior examination data is allocated to the patient or to at least one further one of a plurality of patients. In particular, the selection of the prior examination data is allocated to the patient in case the database comprises at least one prior patient record of the prior examination data which is allocated to the patient, and which is addressing a prior physiological condition of the patient correlated to at least one of the N1 findings, wherein the selection of the prior examination data is the at least one prior patient record.

Each one of the at least one prior patient records allocated to the patient can comprise a prior medical picture and/or a prior final report allocated to the patient and acquired in the frame of a historical examination of the patient. Hence, both the current medical image as well as the at least one prior patient record of the selection of the prior examination data are allocated to the same patient, thus, enabling a direct determination of a possible temporal development of an anatomical structure correlated to a certain one of the N1 findings. In other words, the selection of the prior examination data is allocated to the same patient in case an overlap exists between the certain one of the N1 findings and corresponding historical data of the same patient. Otherwise, the selection of the prior examination data is allocated to the at least one further one of a plurality of patients, hence to at least one different patient than the current one.

According to a further embodiment, in case the selection of the prior examination data is allocated to the patient, the comparison unit is configured to determine a similarity score based on a comparison between each of the N1 fingerprints for the N1 findings and each of at least one fingerprint for the at least one prior patient record allocated to the patient, and wherein, in case the determined similarity score is above a certain threshold level, the respective current pathological condition is equivalent to the prior pathological condition of the patient.

The selection of the prior examination data can comprise prior examination data of a single or of a plurality of prior examinations of the patient. In the latter case, the comparison unit is configured to determine a certain one of the at least one prior patient record allocated to the patient showing the highest similarity with the respective one of the N1 findings. Then, the similarity score is determined between each of the N1 fingerprints for the N1 findings and the certain prior patient record showing the highest similarity with the respective one of the N1 findings. In case the determined similarity score is above a certain threshold level, the respective prior pathological condition can be regarded as still valid at the time of the current examination of the patient. Thus, the respective current pathological condition can be set equivalent to the prior pathological condition. For example, in case, during a prior examination of the patient, a certain anatomical structure has been determined as showing a lung tumor, and the size and/or the shape of a certain one of the N1 findings showing the same anatomical structure is similar to the prior examination, the prior pathological condition of the lung cancer can be upheld. On the other hand, in case the similarity score is below the certain threshold level, the current pathological condition cannot be set equivalent to the prior pathological condition but has to be determined by comparing the respective one of the N1 findings with prior examination data allocated to at least one further one of the plurality of patients. In a similar way, in case the database does either not include prior patient records allocated to the patient, or the prior patient records allocated to the patient do not address an anatomical structure correlated to the respective one of the N1 findings, the respective one of the N1 findings has to be compared with prior examination data allocated to at least one further one of the plurality of patients.

According to a further embodiment, in case the selection of the prior examination data is allocated to the at least one further one of the plurality of patients, the selection of the prior examination data comprises a number N3 of similar patient records allocated to the at least one further one of the plurality of patients, with N3 ≥ 1, wherein the comparison unit is configured to determine at least one probable current pathological condition candidate with a respective similarity score based on a comparison between each of the N1 fingerprints for the N1 findings and all fingerprints of each of the N3 similar patient records, and wherein the current pathological condition is a list of the at least one probable current pathological condition candidate with the respective similarity score. In particular, the list comprises a number N4 of the probable current pathological condition candidates with the highest similarity scores, with N4 ≥ 1.

The N3 of similar patient records can comprise all prior examination data stored within the database which address the respective one of the N1 findings, for example, which are related to the same anatomical structure than the respective one of the N1 findings or which are covering at least a certain pathological region, the respective one of the N1 findings is located as well, wherein a pathological region can be, for example, a region and/or a section of the lung of the patient. In other words, the respective one of the N1 findings of the current examination is matched with all prior examination data stored in the database covering the same pathological region than the respective one of the N1 findings. In case the respective one of the N1 findings matches to a certain degree with a certain anatomical structure of the prior examination data, the respective prior pathological condition is included within the list of possible pathological condition candidates together with the respective similarity score, whereby, the respective similarity score is based on a comparison between the respective one of the N1 fingerprints for the N1 findings and a fingerprint correlated to the certain anatomical structure of the prior examination data. Hereby, the list of possible current pathological candidates can comprise a single or a plurality of possible pathology condition candidates. The list of possible current pathological candidates can be sorted in order to indicate the number N4 of the probable pathological condition candidates with the highest similarity scores. Preferably, the similarity score of each member of the list of probable current pathological condition candidates is compared to a certain threshold. In case the similarity scores of all members of the list of probable current pathological condition candidates are below a certain value, the comparison unit is configured to set the current pathological condition to a healthy state for the respective one of the N1 findings. This way, the comparison unit is capable of providing the most probable pathological condition candidates with respect to the respective one of the N1 findings and, thus, assisting the user in determining a correct diagnosis based on the respective one of the N1 findings.

According to a further embodiment, the graphical user interface is configured to allow a selection among the N4 probable current pathological condition candidates by the user.

The graphical user interface can be configured to provide, after the user selects a certain one of the N1 findings within the current medical image, a list comprising the N4 probable current pathological condition candidates as determined for the certain one of the N1 findings. The graphical user interface is configured to allow the selection of one of the current pathological condition candidates via the user, for example, by clicking on a graphical representation of one of the N4 probable current pathological condition candidates. This way, the user can select one of the N4 probable current pathological condition candidates without the necessity of any further user input in terms of a text input. The selected one of the N4 probable current pathological condition candidates can be stored within the data set and included within the current final report. Unselected ones of the N4 probable current pathological condition candidates can be dismissed and ignored for the further procedure.

According to a further embodiment, the comparison unit is configured to determine the similarity score using a machine learning algorithm. In particular, the machine learning algorithm is configured to be trained continuously with the training data.

In particular, the comparison unit can comprise a deep neural network which can be pretrained in order to correctly identify similarities between two medical images, in particular, between the respective fingerprints of two medical images. Preferably, the machine learning algorithm is configured to be trained continuously with the training data. This way, with the continuous extension of the database and/or the training data during usage of the system, in particular with the current patient record, the precision of the machine learning algorithm can be enhanced continuously during the usage of the suggested system.

According to a further embodiment, the data collector unit is configured to acquire supplementary data corresponding to each of the N1 findings by analyzing an input of the user using the graphical user interface and/or by using a respective specific computer program product allocated to an anatomical location of a respective one of the N1 findings.

In the present context, the supplementary data can be understood as additional information allocated to the respective one of the N1 findings, for example a nodal subtype, a shape of the nodal, etc. The supplementary data can be acquired by the respective specific computer program product allocated to the anatomical location of the respective one of the N1 findings, for example a computer-aided design (CAD) system. For example, in case the respective one of the N1 findings is correlated to the anatomical location of the lung of the patient, the computer program product LungCAD can be used to determine the supplementary data. Alternatively, or additionally, the graphical user interface can be configured to acquire the supplementary data via a user input, wherein the user input can be a text input, a gesture, a content of a speech acquired by a voice recognition system, and/or the like. This way, the system allows the specification of the respective one of the N1 findings using the supplementary data which are assisting the corresponding current pathological condition.

According to a further embodiment, the large language model is configured for generating one mini report for each of the N1 findings. In particular, the graphical user interface is configured to display each of the mini reports allocated to the respective one of the N1 findings within the current medical image.

Each mini report can comprise a textual representation of a certain one of the N1 findings, the respective anatomical location, the respective current pathological condition, a selection of the fingerprint of the certain one of the N1 findings, and/or the supplementary data correlated to the certain one of the N1 findings. Preferably, each mini report additionally comprises an indication whether the respective current pathological condition and/or the certain one of the N1 findings is similar to prior patient records allocated to the patient and possibly an indication of the prior pathological condition and/or the supplementary data correlated to the prior patient records allocated to the patient. The mini report may further address changes of the respective anatomical structure in comparison to the prior patient records. Hence, the large language model is configured for transforming the information of the data set correlated to the certain one of the N1 findings into a textual representation. This way, the user is provided with a short summary addressing the certain one of the N1 findings in a textual manner including relevant information in order to derive a possible diagnosis for the certain one of the N1 findings. For example, a mini report may contain the following text: "*A previously identified lung mass within the left lower lobe has demonstrated significant changes compared to the prior examination. The current measure of the mass is approximately 47 mm in diameter, which represents a 2 mm increase in size since the prior examination. Importantly, the margins of the mass have transitioned from being well-defined to irregular and spiculated*". Preferably, for each of the N1 findings, a single one of the mini reports is generated. Alternatively, for each of the N1 findings, a plurality of the mini reports is generated. Each of the mini reports can be stored in the database. The graphical user interface can be configured to display the current medical image including a visual marking of the N1 findings. The graphical user interface can further be configured to display the respective mini report or mini reports concerning the certain one of the N1 findings in the vicinity of the visual representation of the certain one of the N1 findings. This way, the user can be provided with the current medical image together with an overview over all of the N1 findings and the respective mini reports in a convenient manner.

According to a further embodiment, the system is configured to allow a modification of the data set by allowing a modification of the mini reports by the user using the graphical user interface, and by reading the modified mini reports into the data set by the large language model. In particular, the modification of the mini reports can represent a variation of data within the mini reports and/or an extension of the data within the mini reports.

In case the user is modifying one of the mini reports, the large language model is configured to analyze the modified mini report and to extract the modified data within the modified mini report. The modified data are stored within the data set, and, additionally, enhance the database and/or the training data. The modification of the mini report can occur by editing the text of the mini report by the user and/or by adding new information to the text of the mini report. The modification of data within the mini report may be necessary due to an imprecise or incorrect determination of the current pathological condition and/or the supplementary data correlated to the respective one of the N1 findings. The reading of the modified data and the storing within the database and/or the training data consequently improve the precision of the system in determining the current pathological condition and generating the current final report. In a beneficial manner, over time, the number of times the user needs to edit one of the mini reports is expected to decrease dramatically.

According to a further embodiment, the large language model is configured to generate the current final report based on all mini reports and the selection of the prior examination data.

The current final report can represent a single document containing all information which are relevant concerning the current examination of the patient. It can comprise all of the generated mini reports, information of the selection of the prior examination data, for example, prior pathological conditions, and/or at least a section of the current medical image. This way, after selecting the N1 findings within the current medical image by the user, the system is capable of automatically generating a single document comprising all relevant information concerning the N1 findings, both easing and increasing the precision of the determination of a diagnosis based on the current medical image.

According to a further embodiment, the foundation model, the comparison unit, the data collector unit, and the large language model can be part of a computer program product which is executed on a server and/or on a local computer comprising the graphical user interface and configured to control the medical imaging unit. In particular, the database is stored on the server and/or the local computer.

Any embodiment of the first aspect may be combined with any embodiment of the first aspect to obtain another embodiment of the first aspect.

According to a further aspect, a method for using a system for acquiring a current medical image of a patient and generating a current final report based on the acquired current medical image is provided. The method comprises:
displaying a current medical image as acquired by a medical imaging unit by a graphical user interface,
providing a selection of a number N1 of findings within the acquired current medical image by a user of the system by the graphical user interface,
reading a selection of prior examination data from a database,
comparing the N1 findings with the selection of the prior examination data by a content-based image retrieval system,
determining a current pathological condition for each of the N1 findings by the content-based image retrieval system based on the result of the comparison and the selection of the prior examination data,
summarizing all the current pathological conditions and the selection of the prior examination data into a data set by a data collector unit,
allowing a modification of the data set by the user using the graphical user interface,
generating the current final report based on the data set by a large language model, and
extending, based on the data set, the database and/or training data of the content-based image retrieval system.

According to a further aspect, a computer program product comprising instructions is provided which, when the program is executed by a computer, cause the computer to carry out the above-mentioned method.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the system of the present invention apply mutatis mutandis to the method of the present invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematical illustration of an embodiment of a system for acquiring a current medical image of a patient and generating a current final report;
Fig. 2 shows a schematical illustration of a further embodiment of the system according to Fig. 1;
Fig. 3 shows a schematical illustration of an embodiment of a method for using the system according to Fig. 1 or Fig. 2;
Fig. 4 shows a schematical illustration of a further embodiment of the method according to Fig. 3; and
Fig. 5 shows a schematical illustration of a data flow for generating the current final report using the method according to Fig. 3 or Fig. 4.

In the Figures, like reference numerals designate like or functionally equivalent elements, unless otherwise indicated.

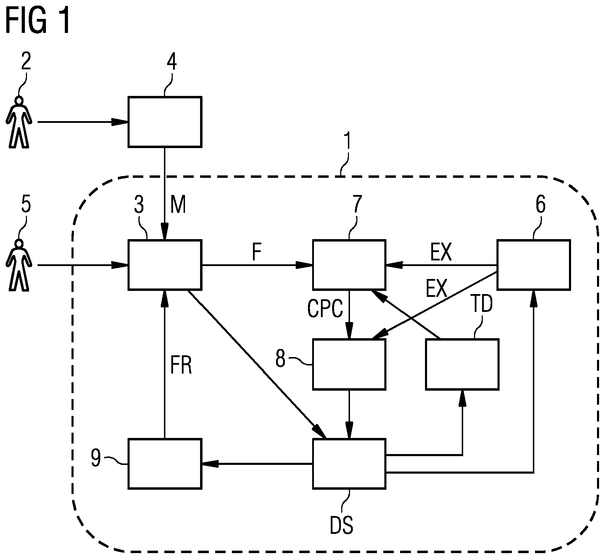

Fig. 1 shows a schematical illustration of a system 1 for acquiring a current medical image M of a patient 2 and generating a current final record FR based on the acquired current medical image M. The system 1 comprises a graphical user interface 3 for displaying the current medical image M. The current medical image M is taken by a medical imaging unit 4 and from there acquired by the system 1. A user 5 of the system 1 can interact with the graphical user interface 3 and select a number N1 of findings F within the acquired current medical image M. The system 1 further comprises a database 6 storing prior examination data. The database 6 can be part of a server or of a local computer. A content-based image retrieval system 7 is configured to compare the N1 findings F within the current medical image M and a selection EX of the prior examination data. The content-based image retrieval system 7 is further configured to determine a current pathological condition CPC for each of the N1 findings F based on the result of said comparison. A data collector unit 8 is configured to summarize all the current pathological conditions CPC as well as the selection EX of the prior examination data into a data set DS. A large language model 9 is configured to generate the current final report FR based on the data set DS. The graphical user interface 3 is configured to display the current final report FR and to allow a modification of the data set DS by the user 5. The system 1 is configured to extend, based on the data set DS, the database 6 and/or training data TD which are used to train the content-based image retrieval system 7. By extending the training data TD in combination with a continuous learning of the content-based image retrieval system 7 using the training data TD, the precision of the content-based image retrieval system 7 in determining all the current pathological conditions CPC is continuously enhanced. Furthermore, the system 1 allows for an automatic generation of the current final report FR without the need of further user inputs, thus saving time and avoiding mistakes in generating the current final report FR. The current final report FR contains all acquired information correlated to the current medical image M, easing a diagnosis for the patient 2 by the user 5.

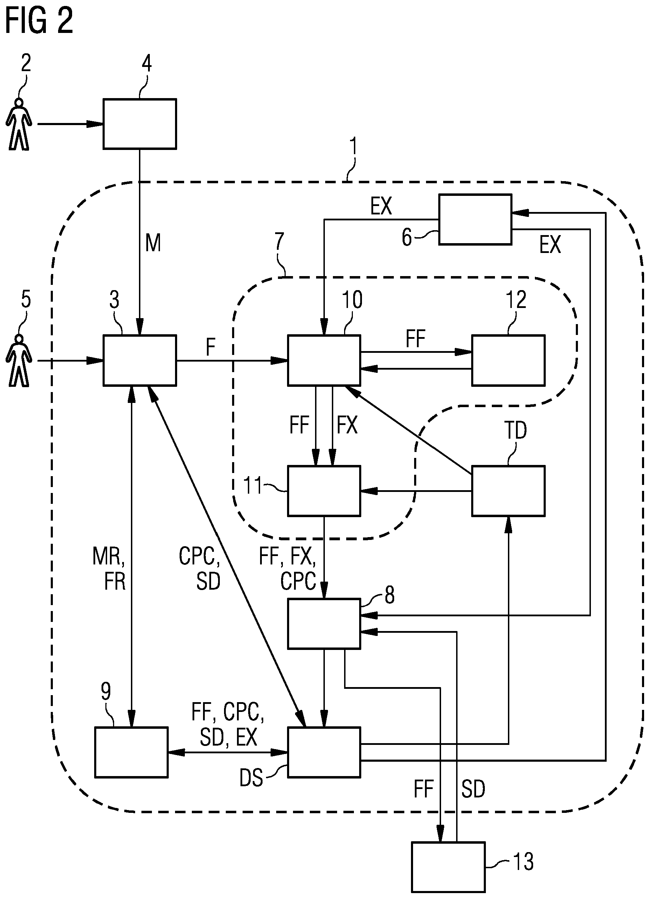

Fig. 2 shows a schematical illustration of a further embodiment of the system 1. For this embodiment, the above-mentioned is relevant as well. Therefore, in the following, only the differences between this embodiment and the first embodiment are addressed.

In this embodiment of the system 1, the content-based image retrieval system 7 comprises a foundation model 10 for determining a number N1 of fingerprints FF for the N1 findings F, with N1 ≥ 1, and for determining a number N2 of fingerprints FX for the selection EX of the prior examination data, with N2 ≥ 1. Hereby, each of the fingerprints FF, FX comprises a vector and/or an array of higher-order information correlated to a certain anatomical structure, for example, the anatomical location of the certain anatomical structure, a length, a diameter, a volume, a mass distribution, or the like. With the fingerprints FF, FX, data correlated to the N1 findings F and the selection EX of the prior examination data can be expressed in a numerical manner. The content-based image retrieval system 7 further comprises a comparison unit 11 for comparing each of the determined fingerprints FF for the N1 findings F with the fingerprints FX for the selection EX of the prior examination data. Hereby, the comparison unit 11 is configured to determine a similarity score between the determined fingerprints FF, FX using a machine learning algorithm, in particular, a deep neural network which is continuously trained with the training data TD. The content-based image retrieval system 7 further comprises an anomaly detector 12 which is configured to detect a possible anomaly of each of the N1 findings F based on the respective one of the N1 fingerprints FF for the N1 findings F. The anomaly detector 12 is configured to detect an anomaly by comparing the respective one of the N1 fingerprints FF for the N1 findings F with predefined and/or continuously trained ranges. In case the anomaly detector 12 does not detect an anomaly concerning a certain one of the N1 fingerprints FF for the N1 findings F, the corresponding current pathological condition CPC is determined as being in a healthy state. A further processing concerning the respective one of the N1 findings F is dismissed, thus saving time. Otherwise, the certain one of the N1 fingerprints FF for the N1 findings F is compared with fingerprints of the prior examination data in order to determine the current pathological condition CPC.

The data collector unit 8 is configured to summarize all the current pathological conditions CPC, all the fingerprints FF, FX, and information of the selection EX of the prior examination data into the data set DS. In case one of the current pathological conditions CPC is a list of possible current pathological condition candidates, the graphical user interface 3 is configured to display said list to the user 5 and to enable a selection of one of the members of said list by the user 5. The data collector unit 8 is further configured to communicate with a CAD computer program product 13 for acquiring supplementary data SD for each of the N1 findings F. The CAD computer program product 13 can be part of the system 1 or be designed as an external computer program product. In order to acquire the supplementary data SD, the CAD computer program product 13 is provided with the fingerprint FF of the respective one of the N1 findings F, in particular with the corresponding anatomical location. Hence, the CAD computer program product 13 can be chosen specifically according to the anatomical location and be used to acquire the supplementary data SD. Additionally, or alternatively, the supplementary data SD can be entered by the user 5 using the graphical user interface 3 in the manner of a text input, a speech recognition, and/or the like. The supplementary data SD comprise additional information correlated to a specific one of the N1 findings F, for example a size of the respective one of the N1 findings F, whether a shape of the respective one of the N1 findings F is rounded or speculated, whether a margin is irregular or well-defined, and/or the like.

The large language model 9 is configured to generate one mini report MR for each of the N1 findings F, based on the respective current pathological condition CPC, the fingerprint FF of the respective one of the N1 findings F, the respective supplementary data SD, as well as information of the selection EX of the prior examination data. Each of the mini reports MR is written in a textual manner and comprises all information concerning a current status as well as a possible historical development of the respective one of the N1 findings F, thus easing a diagnosis of the respective one of the N1 findings F by the user 5. The graphical user interface 3 is configured to display each of the mini reports MR in vicinity of the respective one of the N1 findings F within the current medical image M. The graphical user interface 3 is further configured to allow a modification of each of the mini reports MR by the user 5. The large language model 9 is configured to read the modified mini report MR, to extract the modified information within the respective mini report MR, and to update the information within the data set DS according to the modification by the user 5. Hereby, the modification of the mini report MR can both be an editing of information within one of the mini reports MR and/or an adding additional information to one of the mini reports MR. The large language model 9 is further configured to generate the current final report FR based on all the mini reports MR as well as the information of the selection EX of the prior examination data.

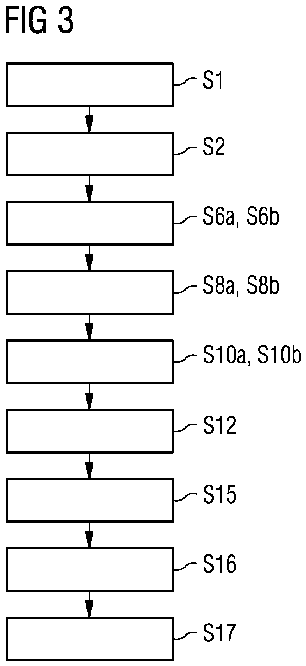

Fig. 3 shows a schematical illustration of an embodiment of a method for using the system 1.

In a first step S1, the current medical image M as acquired by the medical imaging unit 4 is displayed to the user 5 by the graphical user interface 3. In a second step S2, the selection of the N1 findings F within the acquired current medical image M is provided to the user 5 using the graphical user interface 3. In a further step S6a, S6b, the selection EX of the prior examination data is read from the database 6. In a further step S8a, S8b, the N1 findings F are compared with the selection EX of the prior examination data by the content-based image retrieval system 7. In a further step S10a, SlOb, the current pathological condition CPC for each of the N1 findings F is determined by the content-based image retrieval system 7 based on the result of the comparison and the selection EX of the prior examination data. In a further step S12, all the current pathological conditions CPC and the information of the selection EX of the prior examination data is summarized into the data set DS by the data collector unit 8. In a further step S15, the modification of the data set DS by the user 5 is enabled using the graphical user interface 3. In a further step S16, the current final report FR is generated based on the data set DS by the large language model 9. In a further step S17, the database 6 and/or the training data TD of the content-based image retrieval system 7 are extended, based on the data set DS.

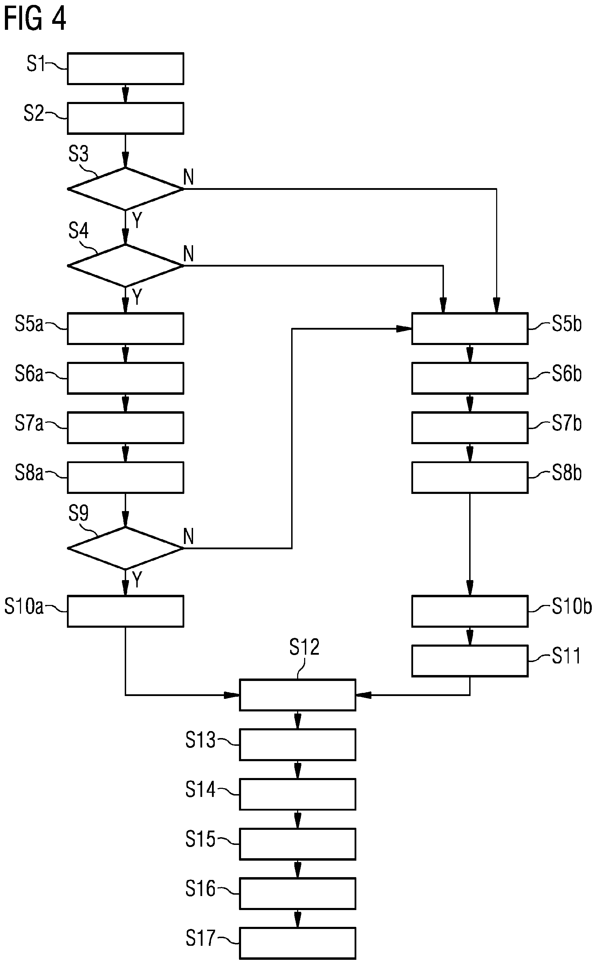

Fig. 4 shows a schematical illustration of a further embodiment of the above-mentioned method. The illustration in Fig. 4 can also be understood as a data flow during the usage of the system 1.

In a first step S1, the current medical image M as acquired by the medical imaging unit 4 is displayed to the user 5 by the graphical user interface 3. In a second step S2, the graphical user interface 3 provides the selection of the number N1 of findings F within the acquired current medical image M by the user 5. In a third step S3, the foundation model 10 determines whether the database 6 comprises at least one prior patient record allocated to the patient 2, for example, acquired during a prior examination of the patient 2 with the system 1. In case the database 6 includes such at least one prior patient record, in a fourth step S4, the foundation model 10 is configured to determine whether said at least one prior patient record is addressing the same anatomical location and/or the same anatomical structure than the respective one of the N1 findings F. In case the determination verifies the existence of such at least one prior patient record, the respective one of the N1 findings F is compared with the respective at least one prior patient record and the method is continued in a further step S5a. In case the database 6 does not include a prior patient record which is both allocated to the patient 2 and addressing the same anatomical location and/or the same anatomical structure than the respective one of the N1 findings F, the respective one of the N1 findings F is compared with prior examination data allocated to at least one further one of a plurality of patients and the method is continued in step S5b.

In step S5a, the at least one prior patient record allocated to the patient 2 is set as the selection EX of the prior examination data. In step S6a, the at least one prior patient record is read from the database 6 by the foundation model 10. In step S7a, the fingerprint FF of each of the N1 findings F is determined by the foundation model 10. In addition, the fingerprints FX for the selection of the prior examination data are determined by the foundation model 10 based on the at least one prior patient record. In step S8a, the fingerprints FF, FX are compared with one another by the comparison unit 11. A respective similarity score is derived representing the degree of similarity between each of the fingerprints FF for of the N1 findings F and the fingerprints FX for the at least prior patient record. In step S9, the comparison unit 11 determines whether the similarity score between the fingerprints FF and FX is higher than a certain threshold level. The threshold level is set in a way to enable the determination whether the respective anatomical structure associated with the respective one of the N1 findings F is similar to a historical state of the same anatomical structure as captured within the prior patient record or whether the respective anatomical structure has changed in size, shape, etc. in the meantime.

In case the similarity score is above the certain threshold level, in step S10a, the current pathological condition CPC of the respective one of the N1 findings F is set to the prior pathological condition as stored within the prior patient record. In other words, in this case, the pathological condition of the respective one of the N1 findings F remains unchanged and the method is continued in step S12. Nonetheless, in case the similarity score is lower than the certain threshold level, hence, in case the respective anatomical structure has significantly changed in terms of size, shape, etc. in comparison to the prior patient record, the respective one of the N1 findings F is compared with prior examination data allocated to the at least one further one of the plurality of patients and the method is continued in step S5b.

In step S5b, prior examination data allocated to at least one further one of a plurality of patients is set as the selection EX of prior examination data. In step S6b, the selection EX of prior examination data is read by the foundation model 10. In step S7b, the fingerprint FF of each of the N1 findings F is determined by the foundation model 10. In addition, the fingerprints FX for the selection of the prior examination data is determined by the foundation model 10 based on prior examination data allocated to the at least one further one of the plurality of patients. In step S8b, the fingerprints FF, FX are compared with one another by the comparison unit 11. A respective similarity score is derived representing the degree of similarity between each of the fingerprints FF for of the N1 findings F and the fingerprints FX for the selection EX of the prior examination data. In case the selection EX of prior examination data comprises a plurality of records allocated to at least one further patient, the fingerprint FF of each of the N1 findings F is compared with each fingerprint FX of the respective record. Each comparison provides at least one current pathological condition candidate with a respective similarity score.

In step SlOb, the current pathological condition CPC is set to the number of current pathological condition candidates, hence, the current pathological condition CPC represents a list of at least one current pathological condition candidates with the respective similarity score. Said list can be sorted in order to identify and to provide the number N5 of most probable current pathological condition candidates with N5 ≥ 1.

In step S11, a selection of one of the members of the N5 probable current pathological condition candidates is provided to the user 5 using the graphical user interface 3. Additionally, or alternatively, said selection can be performed automatically using the most probable current pathological condition candidate with the highest similarity score. It is further feasible to inform the user 5 in case the list of N5 probable current pathological condition candidates comprises more than one member. Hence, a certain uncertainty is given in determining the list of current pathological condition candidates, followed by the manual selection of one of the current pathological condition candidates by the user 5. The method is continued in step S12.

In step S12, all the information correlated to the N1 findings F are summarized into the data set DS by the data collector unit 8. Said information comprise at least the fingerprint FF for each of the N1 findings F, the fingerprints FX for the selection EX of the prior examination data, and all the current pathological conditions CPC.

In step S13, the supplementary data SD are acquired by the data collector unit 8 and stored in the data set DS. Note, that step S13 can be skipped in case no supplementary data SD is entered and/or required for the respective one of the N1 findings. Hereby, the supplementary data SD can be acquired using the CAD computer program product 13 and/or the user input using the graphical user interface 3.

In step S14, for each of the N1 findings F, one mini report MR is generated by the large language model 9 and displayed to the user 5 by the graphical user interface 3. In step S15, the modification of each of the mini reports MR is provided to the user 5 by the graphical user interface 3. Possible modifications are read, and the modified information are extracted and stored into the data set DS by the large language model 9. In step S16, the large language model 9 generates the current final report FR based on all of the mini reports MR and information of the prior examination data. Hence, all the information of the data set DS is transformed by the large language model 9 into a textual representation. The current final report FR is displayed to the user 5 by the graphical user interface 3. Additionally, the current final report FR is stored for archiving purposes within the database 6. In step S17, the information of the data set DS is used to extend the training data TD which are used to train the foundation model 10 and/or the comparison unit 11, more specifically to train the machine learning algorithms within the foundation model 10 and/or the comparison unit 11, and/or to extend the database 6. In this way, the database 6 and the training data TD are updated and extended continuously during the usage of the system 1. The precision of the foundation model 10 regarding the determination of the fingerprints FF, FX as well as of the comparison unit 11 regarding the determination of the current pathological condition CPC, is continuously improved.

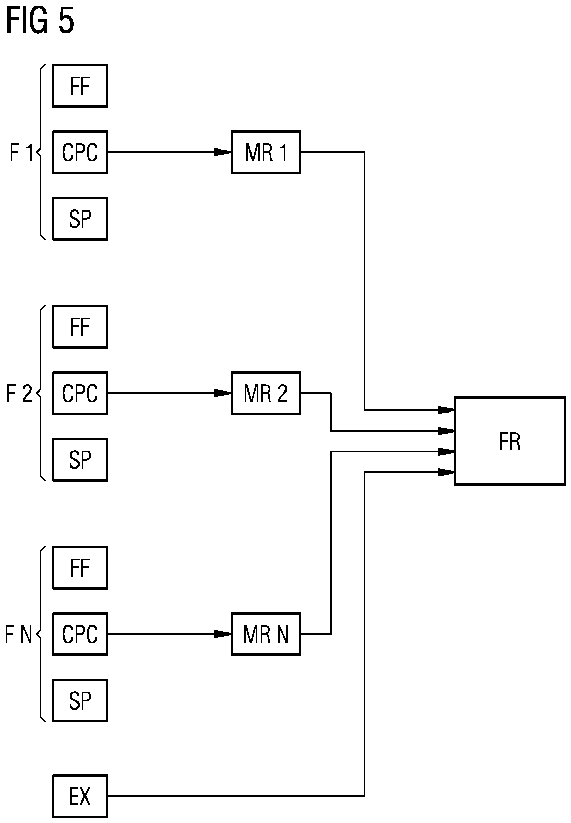

Fig. 5 shows a schematical illustration of a data flow for generating the current final report FR using the above-mentioned method.

For each one of the N1 findings (indicated in Fig. 5 as F 1, F 2, ..., F N) the respective fingerprint FF for the N1 findings F, the current pathological condition CPC, and the supplementary data SD is determined. Said data are used for generating each one mini report MR for each one of the N1 findings (indicated in Fig. 5 as MR 1, MR 2, ..., MR N). Eventually, all the mini reports MR plus information of the selection EX of the prior examination data are used to generate the current final report FR.

Although the present invention has been described in accordance with preferred embodiments, it is obvious for the person skilled in the art that modifications are possible in all embodiments.

## Claims

1. A system (1) for acquiring a current medical image (M) of a patient (2) and generating a current final report (FR) based on the acquired current medical image (M), the system (1) comprising:
a graphical user interface (3) for receiving and displaying the current medical image (M) as acquired by a medical imaging unit (4), and for providing a selection of a number N1 of findings (F) within the acquired current medical image (M) by a user (5) of the system (1), with N1 ≥ 1,
a database (6) storing prior examination data,
a content-based image retrieval system (7) for comparing the N1 findings (F) with a selection (EX) of the prior examination data, and for determining a current pathological condition (CPC) for each of the N1 findings (F) based on a result of the comparison and the selection (EX) of the prior examination data,
a data collector unit (8) for summarizing all the current pathological conditions (CPC) and the selection (EX) of the prior examination data into a data set (DS), and
a large language model (9) for generating the current final report (FR) based on the data set (DS),
wherein the graphical user interface (3) is configured to allow a modification of the data set (DS) by the user (5), and
wherein the system (1) is configured to extend, based on the data set (DS), the database (6) and/or training data (TD) of the content-based image retrieval system (7).

2. The system according to claim 1,
further comprising an anomaly detector (12) for detecting an anomaly within each of the N1 findings (F), in particular, the anomaly detector (12) is configured to set the respective current pathological condition (CPC) to a healthy state in case no anomaly is detected.

3. The system according to claim 1 or 2,
wherein the content-based image retrieval system (7) comprises a comparison unit (11) for comparing the N1 findings (F) with the selection (EX) of the prior examination data.

4. The system according to claim 3,
wherein the content-based image retrieval system (7) comprises a foundation model (10) for determining a number N1 of fingerprints (FF) for the N1 findings (F), and for determining a number N2 of fingerprints (FX) for the selection (EX) of the prior examination data, with N2 ≥ 1, and
wherein the comparison unit (11) is configured to compare the N1 fingerprints (FF) for the N1 findings (F) with the N2 fingerprints (FX) for the selection (EX) of the prior examination data.

5. The system according to claim 4,
wherein the selection (EX) of the prior examination data is allocated to the patient (2) or to at least one further one of a plurality of patients,
in particular, the selection (EX) of the prior examination data is allocated to the patient (2) in case the database (6) comprises at least one prior patient record of the prior examination data which is allocated to the patient (2), and which is addressing a prior physiological condition of the patient (2) correlated to at least one of the N1 findings (F), wherein the selection (EX) of the prior examination data is the at least one prior patient record.

6. The system according to claim 5,
wherein, in case the selection (EX) of the prior examination data is allocated to the patient (2), the comparison unit (11) is configured to determine a similarity score based on a comparison between each of the N1 fingerprints (FF) for the N1 findings (F) and each of at least one fingerprint for the at least one prior patient record allocated to the patient, and
wherein, in case the determined similarity score is above a certain threshold level, the respective current pathological condition (CPC) is equivalent to the prior pathological condition of the patient (2).

7. The system according to claim 5,
wherein, in case the selection (EX) of the prior examination data is allocated to the at least one further one of the plurality of patients, the selection (EX) of the prior examination data comprises a number N3 of similar patient records allocated to the at least one further one of the plurality of patients, with N3 ≥ 1,
wherein the comparison unit (11) is configured to determine at least one probable current pathological condition candidate with a respective similarity score based on a comparison between each of the N1 fingerprints (FF) for the N1 findings (F) and all fingerprints of each of the N3 similar patient records, and
wherein the current pathological condition (CPC) is a list of the at least one probable current pathological condition candidate with the respective similarity score,
in particular, the list comprises a number N4 of the probable current pathological condition candidates with the highest similarity scores, with N4 ≥ 1.

8. The system according to claim 7,
wherein the graphical user interface (3) is configured to allow a selection among the N4 probable current pathological condition candidates by the user (5).

9. The system according to one of claims 6 - 8,
wherein the comparison unit (11) is configured to determine the similarity score using a machine learning algorithm, in particular, the machine learning algorithm is configured to be continuously trained with the training data (TD).

10. The system according to one of claims 1 - 9,
wherein the data collector unit (8) is configured to acquire supplementary data (SD) corresponding to each of the N1 findings (F) by analyzing an input of the user (5) using the graphical user interface (3) and/or by using a respective specific computer program product allocated to an anatomical location of a respective one of the N1 findings (F).

11. The system according to one of claims 1 - 10,
wherein the large language model (9) is configured for generating one mini report (MR) for each of the N1 findings (F), in particular, the graphical user interface (3) is configured to display each of the mini reports (MR) allocated to the respective one of the N1 findings (F) within the current medical image (M).

12. The system according to claim 11,
wherein the system (1) is configured to allow a modification of the data set (DS)
a) by allowing a modification of the mini reports (MR) by the user (5) using the graphical user interface (3), and
b) by reading the modified mini reports (MR) into the data set (DS) by the large language model (9),
in particular, the modification of the mini reports (MR) represents a variation of data within the mini reports (MR) and/or an extension of the data within the mini reports (MR).

13. The system according to claim 11 or 12,
wherein the large language model (9) is configured to generate the current final report (FR) based on all mini reports (MR) and the selection (EX) of the prior examination data.

14. The system according to one of claims 1 - 13,
wherein the foundation model (10), the comparison unit (11), the data collector unit (8), and the large language model (9) are part of a computer program product which is executed on a server and/or on a local computer comprising the graphical user interface (3) and configured to control the medical imaging unit (4), in particular, the database (6) is stored on the server and/or the local computer.

15. A method for using a system (1) for acquiring a current medical image (M) of a patient (2) and generating a current final report (FR) based on the acquired current medical image (M), the method comprising:
displaying (S1) the current medical image (M) as acquired by a medical imaging unit (4) by a graphical user interface (3),
providing (S2) a selection of a number N1 of findings (F) within the acquired current medical image (M) by a user (5) of the system (1) by the graphical user interface (3),
reading (S6a, S6b) a selection (EX) of prior examination data from a database (6),
comparing (S8a, S8b) the N1 findings (F) with the selection (EX) of the prior examination data by a content-based image retrieval system (7),
determining (S10a, SlOb) a current pathological condition (CPC) for each of the N1 findings (F) by the content-based image retrieval system (7) based on the result of the comparison and the selection (EX) of the prior examination data,
summarizing (S12) all the current pathological conditions (CPC) and the selection (EX) of the prior examination data into a data set (DS) by a data collector unit (8),
allowing (S15) a modification of the data set (DS) by the user (5) using the graphical user interface (3),
generating (S16) the current final report (FR) based on the data set (DS) by a large language model (9), and
extending (S17), based on the data set (DS), the database (6) and/or training data (TD) of the content-based image retrieval system (7).

16. A computer program product comprising instructions which, when the program is executed by a computer, cause the computer to carry out the method of claim 15.
